# EUROPEAN PATENT APPLICATION

(11) **EP 4 079 312 A1**
(43) Date of publication of application: **26.10.2022**
(21) Application number: 21169770.1
(22) Date of filing: 22.04.2021
(51) Int. Cl.: A61K 35/17, A61P 31/04

(54) **NEW TREATMENT OF SEPSIS**

(71) Applicant: Guizhou Sinorda Biotechnology CO., Ltd., Guiyang, Guizhou 550008 (CN)
(72) Inventor: YANG, Yuan, Guiyang (CN); TANG, Jingling, Guiyang (CN); HU, Pingsheng, Guiyang (CN); LI, Xiaoyang, Guiyang (CN); WU, Liyun, Guiyang (CN)
(74) Representative: Zacco Sweden AB

(57) **Abstract**

The present invention relates to a method for treatment of sepsis in a subject comprising administering a pharmaceutical composition comprising an effective amount of allogeneic Natural Killer cells to said subject, and to methods for predicting a subject's susceptibility to such method of treatment.

## Description

### Technical field

The present invention relates to the field of therapeutic treatment of human and animal subjects, and in particular to methods using allogeneic cells in treatment of sepsis.

### Background

Sepsis is an extremely dangerous condition that is caused by the body's response to an infection or an injury. Blocking inflammatory mediators or pathogen recognition signaling pathways often failed, and immunological modification therapies could be new approaches using in sepsis.

During the process of sepsis the immune system is over-activated, producing excessive levels of cytokines, signaling molecules that attract immune cells. Elevated levels of those cells secrete more cytokines, and this "cytokine storm" recruits even more immune cells, fueling a vicious cycle. Instead of stopping the initial infection, immune factors attack the body's tissues and organs, causing organ failure and death. No clinical trials of interventions targeting cytokine production or effects have so far been successful (Chousterman et al., 2017). Even if patients survive in the hyper-inflammatory phase, the subsequent immunosuppressive phase is still life threatening.

Allogeneic Natural Killer ("NK") cells have been suggested for use in adoptive immunotherapy for the treatment of cancer (Geller et al., 2011). Infusion of allogeneic NK cells after *ex vivo* expansion has been reported as largely safe (Lim et al., 2015).

### Summary of the invention

The present invention aims to provide novel therapeutic treatments of sepsis.

In a first aspect, the present invention relates to a method for treatment of sepsis in a subject comprising administering a pharmaceutical composition comprising an effective amount of allogeneic Natural Killer cells to said subject.

In one embodiment, the administering of allogeneic Natural Killer cells is performed through multiple administrations.

In one embodiment, the allogeneic Natural Killer cells are administered by at least one intravenous infusion.

In one embodiment, at least 10⁹ allogeneic Natural Killer cells are administered per administration.

In one embodiment, the subject is immunocompromised.

In one embodiment, the subject suffers from sepsis caused by a microbial infection.

In one embodiment, least 70% of the cells in the pharmaceutical composition are CD3⁻ and CD56⁺.

In one embodiment, at least 70% of the cells in the pharmaceutical composition are CD56⁺ and CD16⁺.

In one aspect, the invention relates to allogeneic Natural Killer cells for use in a method according to the above described aspect of the invention.

In one aspect, the invention relates to a use of allogeneic Natural Killer cells in the manufacture of a pharmaceutical composition for use in a method according to the above described aspect of the invention.

In one aspect, the invention relates to a method for predicting a subject's susceptibility to a method of treatment according to the above described aspect of the invention, comprising co-incubating serum from the subject with allogeneic Natural Killer cells and monitoring the level of interleukin-6 in said serum over time, wherein a reduction of the level of interleukin-6 over time is indicative of the subject's susceptibility to the method of treatment.

In one aspect, the invention relates to a method for predicting a subject's susceptibility to a method of treatment according to the above described aspect of the invention, comprising co-incubating serum from the subject with allogeneic Natural Killer cells and measuring proportional expression of at least one cell surface marker protein on the cell surface of said allogeneic Natural Killer cells before and after co-incubation with the serum, wherein a reduced proportional expression of the at least one cell surface marker protein on the cell surface of said allogeneic Natural Killer cells, wherein the at least one cell surface marker protein is selected from the group consisting of CD56, CD16, DNAM-1, NKG2D, and NKP30/NKP46, is indicative of the subject's susceptibility to the method of treatment,.

In one aspect, the invention relates to a method for predicting a subject's susceptibility to a method of treatment according to the above described aspect of the invention, comprising
- co-incubating serum from the subject with allogeneic Natural Killer cells and monitoring the level of interleukin-6 in said serum over time; and
- co-incubating serum from the subject with allogeneic Natural Killer cells and measuring proportional expression of at least one cell surface marker protein on the cell surface of said allogeneic Natural Killer cells before and after co-incubation with the serum, wherein a reduced proportional expression of the at least one cell surface marker protein on the cell surface of said allogeneic Natural Killer cells, wherein the at least one cell surface marker protein is selected from the group consisting of CD56, CD16, DNAM-1, NKG2D, and NKP30/NKP46;
wherein a reduction of the level of interleukin-6 over time in combination with a reduced proportional expression of the at least one cell surface marker protein on the cell surface of said allogeneic Natural Killer cells are indicative of the subject's susceptibility to the method of treatment.

In one embodiment of this aspect, the serum and the allogeneic Natural Killer cells are co-incubated for a period of time of 16-24 hours.

### Definitions

For the purposes of this disclosure "Natural Killer cells", or "NK cells", is defined as a subset of lymphocytes capable especially of destroying tumor cells or pathogens without prior exposure to the target cell and without having it presented with a histocompatibility antigen. The characteristic surface markers of NK cells are CD45+, CD3-,CD56+.

### Detailed description

The present invention is based on the finding that adoptive transfusion of allogeneic NK cells can be used for the treatment of sepsis patients. NK cell therapy can reduce the level of inflammatory cytokines in patients, and can increase anti-inflammatory cytokines simultaneously. The treatment could help patients improve their ability to fight infection.

In a first aspect, the present invention relates to a method for treatment of sepsis in a subject comprising administering a pharmaceutical composition comprising an effective amount of allogeneic Natural Killer cells to said subject.

Purification and expansion of Natural Killer cells is generally known in the art and may be performed according to established methods. Exemplary protocols for purification and expansion of NK cells is provided in the Examples.

The pharmaceutical composition is preferably suitable for intravenous infusion into a subject of interest, such as a human subject. Suitable pharmaceutical compositions comprise water for injection and optionally human albumin and other pharmaceutically acceptable excipients. Compositions for intravenous injection should be pyrogen-free and with a suitable pH, isotonicity and stability. Suitable compositions may make use of, for example, isotonic vehicles such as sodium chloride injection, Ringer's injection or Lactated Ringer's injection. Preservatives, stabilisers, buffers, antioxidants and/or other additives may be included, as required. Formulation of suitable pharmaceutical compositions is furthermore known in the art, e.g. through Remington, The Science and Practice of Pharmacy, 23rd ed. (Elsevier, Inc. 2020, ISBN 978-0-12-820007-0).

The administration of the composition comprising allogeneic Natural Killer cells is generally performed through a single or multiple administrations, such as at least one intravenous infusion. In one embodiment administration is performed through at least two administrations, at least three administrations, at least four administrations, at least five administrations, at least six administrations, at least seven administrations, at least eight administrations, at least nine administrations, or at least ten administrations. In one embodiment, at least 10⁹ allogeneic Natural Killer cells are administered per administration.

In some embodiments, the subject is immunocompromised. In some embodiments the subject is immunocompromised due to ongoing pharmaceutical treatment of other conditions, such as an ongoing treatment of a cancer or an autoimmune disorder, or due to an organ transplant. In some embodiments the subject is immunocompromised due to a disease or a dysfunction caused or complicated by a disease. Immunocompromised patients may not respond appropriately to standard sepsis therapies, as disclosed in the examples of the present disclosure. The methods according to the invention may therefore be of significant advantage to this patient population.

In some embodiments, the subject suffers from sepsis caused by a microbial infection, such as a bacterial infection, a viral infection, a fungal infection, or any combination thereof.

In some embodiments at least 70% of the cells in the pharmaceutical composition are CD3⁻ and CD56⁺. In some embodiments, at least 70% of the cells in the pharmaceutical composition are CD56⁺ and CD16⁺.

In one aspect, the invention relates to allogeneic Natural Killer cells for use in a method according to the above described aspect of the invention.

In one aspect, the invention relates to a pharmaceutical composition comprising allogeneic Natural Killer cells, as described above, for use in a method according to the above described aspect of the invention.

In one aspect, the invention relates to a use of allogeneic Natural Killer cells in the manufacture of a pharmaceutical composition for use in a method according to the above described aspect of the invention. Suitable pharmaceutical compositions comprise water for injection and optionally human albumin and other pharmaceutically acceptable excipients. Compositions for intravenous injection should be pyrogen-free and with a suitable pH, isotonicity and stability. Suitable compositions may make use of, for example, isotonic vehicles such as sodium chloride injection, Ringer's injection or Lactated Ringer's injection. Preservatives, stabilisers, buffers, antioxidants and/or other additives may be included, as required. Formulation of suitable pharmaceutical compositions is furthermore known in the art, e.g. through Remington, The Science and Practice of Pharmacy, 23rd ed. (Elsevier, Inc. 2020, ISBN 978-0-12-820007-0).

Furthermore, biomarkers useful in predicting treatment effectiveness are provided. Blood plasma from a prospective patient is co-incubated with NK cells. If the NK cells reduce IL6 levels in the blood plasma and/or reduce markers of killing function of NK cells, this indicates that NK cell therapy may be effective in treatment of the patient.

Thus, in one aspect, the invention relates to a method for predicting a subject's susceptibility to a method of treatment according to the above described aspect of the invention, comprising co-incubating serum from the subject with allogeneic Natural Killer cells and monitoring the level of interleukin-6 in said serum over time, wherein a reduction of the level of interleukin-6 over time is indicative of the subject's susceptibility to the method of treatment.

In one aspect, the invention relates to a method for predicting a subject's susceptibility to a method of treatment according to the above described aspect of the invention, comprising co-incubating serum from the subject with allogeneic Natural Killer cells and measuring proportional expression of at least one cell surface marker protein on the cell surface of said allogeneic Natural Killer cells before and after co-incubation with the serum, wherein the at least one cell surface marker protein is selected from the group consisting of CD56, CD16, DNAM-1, NKG2D, and NKP30/NKP46, and wherein a reduced proportional expression of the at least one cell surface marker protein on the cell surface of said allogeneic Natural Killer cells is indicative of the subject's susceptibility to the method of treatment,.

In one aspect, the invention relates to a method for predicting a subject's susceptibility to a method of treatment according to the above described aspect of the invention, comprising
- co-incubating serum from the subject with allogeneic Natural Killer cells and monitoring the level of interleukin-6 in said serum over time; and
- co-incubating serum from the subject with allogeneic Natural Killer cells and measuring proportional expression of at least one cell surface marker protein on the cell surface of said allogeneic Natural Killer cells before and after co-incubation with the serum, wherein the at least one cell surface marker protein is selected from the group consisting of CD56, CD16, DNAM-1, NKG2D, and NKP30/NKP46;
wherein a reduction of the level of interleukin-6 over time in combination with a reduced proportional expression of the at least one cell surface marker protein on the cell surface of said allogeneic Natural Killer cells are indicative of the subject's susceptibility to the method of treatment.

In one embodiment of this aspect, the serum and the allogeneic Natural Killer cells are co-incubated for a period of time of 16-24 hours.

The following examples are included as illustrations of the present invention and shall not be construed as limiting the scope of the invention, which is as defined in the appended claims.

### Examples

### Example 1

A 58 years male patient was admitted to the inventors' medical clinic with advanced lung cancer. He received a variety of anti-tumor treatments, including radiation, chemotherapy and immunotherapy, but the tumor continued to progress rapidly. Multiple treatments had resulted in severe liver and kidney dysfunction, impaired immunity, and simultaneous fungal and bacterial infections. These complications prevented the patient from continued anti-tumor therapy. The patient needed plasmapheresis and hemodialysis and required intensive care. Antibiotic treatment failed to control the infection.

Sepsis biomarkers including circulating C-reactive protein (CRP), procalcitonin (PCT) and serum IL-6 concentrations were elevated. The patient was delirious.

After consensus with the patient and family, the patient received an infusion of expanded allogeneic Natural Killer ("NK") cells obtained from the research laboratory in the hospital. Flow cytometry was used to identify NK cells through the characteristic cell surface markers CD45+, CD3-,CD56+. NK cells were cultured from peripheral blood monocyte cells of healthy donor. After stimulation with cytokines, such as IL2, IL7 and IL15, the NK cells were polarized and expanded by a factor of 300-500. The purity of NK cells (CD3-CD56+) in the product was 78.3%, and the activation rate (CD56+CD16+) was 74.2% of NK cells.

The patient received seven administrations by intravenous infusion of allogeneic NK cells with 10⁹-2^{∗}10⁹ cell count per infusion. After NK cell infusion, circulating sepsis biomarkers decreased significantly (Table 1), and the patient's body temperature decreased, subjective feeling improved, and consciousness improved.

**Table 1: Change of sepsis biomarkers**

| **NK cell infusi on times** | **IL6 (fold of normal)** | | **Procalcitonin (fold of normal)** | | **C-reactive protein (fold of normal)** | | **D-Dimer (fold of normal)** | |
|---|---|---|---|---|---|---|---|---|
| | **Before infusion** | **After infusion** | **Before infusion** | **After infusion** | **Before infusion** | **After infusion** | **Before infusion** | **After infusion** |
| 1^{st} | 17.146 | 8.88 | 107 | 59 | 21.28 | 11.17 | 7.28 | 5.54 |
| 2^{nd} | 8.88 | 20.66 | 59 | 48.8 | 11.17 | 7.13 | 5.54 | 4.55 |
| 3^{rd} | 163.29 | 12.84 | 66.2 | 63.4 | 19.84 | 17.44 | 6.66 | 4.17 |
| 4^{th} | 16.54 | 7.90 | 38.6 | 46.6 | 10.17 | 7.42 | 3.19 | 2.6 |
| 5^{th} | 309.00 | 24.51 | 107.4 | 107 | 17.10 | 35.11 | 4.8 | 2.2 |
| 6th | 24.51 | 8.13 | 107 | 68.6 | 35.11 | 14.87 | 2.2 | 2.57 |
| 7th | 55.75 | 4.32 | 86.6 | 91.2 | 13.28 | 16.55 | 3.81 | 2.13 |

Serum cytokines concentration were measured before and after NK cell therapy. Data showed that treatment reduced serum levels of inflammatory cytokines (IL6,IL10,IL8 and IL-1RA) and increased levels of anti-inflammatory cytokines (IFN-γ and TNF-α) (Table 2).

**Table 2: Change of serum cytokines before and after NK cell therapy**

| **Cytokine (pg/mL)** | **Pre-treatment** | **Post-treatment** | **Function** |
|---|---|---|---|
| IL-6 | 134.8 | 10.9 | Immune suppression |
| IL-10 | 49.8 | 0.7 | Immune suppression |
| IL-8 | 67.7 | 29.3 | Immune suppression |
| IL-1RA | 11255 | 1055 | Immune suppression |
| TNF-α | 7.9 | 9.6 | Immune activation |
| IFN-γ | 74.1 | 128.9 | Immune activation |

After three NK cell infusions in 24 hours, the percentage of NK cell subset in the patient's blood was analysed. We subdivided the peripheral blood NK cell subsets of patients, and the data showed that the proportion of NK cell subsets associated with killing function (CD56+CD16+) increased from 8.1% to 16.8%. Besides, NK cell treatment also resulted in increased expression of cell surface markers DNAM-1, NKG2D, and NKP30/NKP46 related to NK cell killing (Table 2).

**Table 3: Change of NK subset/surface markers in peripheral blood of patient**

| **NK cell subset/surface markers** | **Pre-treatment** | **Post-treatment** | **Function** |
|---|---|---|---|
| Cytotoxic subset (CD56+CD16+) | 8.12 | 16.80 | Cytotoxicity |
| Negative subset (CD56-CD16-) | 46.40 | 35.00 | Unknown¹ |
| DNAM-1 | 8.14 | 22.6 | Cytotoxicity |
| NKG2D | 46.80 | 58.30 | Cytotoxicity |
| NKP30/NKP46 | 6.99 | 15.90 | Cytotoxicity |

| | | | |
|---|---|---|---|
| ¹ (Björkström et al., 2010) | | | |

### Example 2

In order to demonstrate the therapeutic effect of NK cells *in vitro,* the patients' serum was co-incubated for 16-24 hours. These cells were plated in 96-well U bottom plate and maintained in a humidified atmosphere containing 5 % CO₂ at 37 °C. We added 1^{∗}10⁵ NK cells and 50 µL of the patient's serum together in one well in a 96-well plate. Serum was used as control. We evaluated the change of IL-6 concentration in the culture medium. NK cells decreased the IL-6 level in the serum (Table 4).

**Table 4: Change of IL6 in the co-culture system**

| **Samples** | **IL6 (pg/mL) Pre-treatment** | **IL6 (pg/mL) Post-treatment** |
|---|---|---|
| NK1 | 118.02+2.14 | 103.61+2.11 |
| NK2 | 118.02+2.14 | 98.06±4.00 |
| NK3 | 118.02+2.14 | 91.10±3.82 |

The testing has been conducted on three different batches of NK cell products (NK1, NK2 and NK3), expanded from three different healthy donors.

The change in expressed cell surface markers of NK cells in the co-culture system was tested using the same testing panel for NK cell surface markers CD56, CD16, DNAM-1, NKG2D, and NKP30/NKP46.

Interestingly, change in expression of cell surface markers differ between therapeutic use i*n vivo* and co-culture with serum *in vitro.* After treatment with patient plasma, the killing function of NK cells was impaired, which was manifested as the proportion of cytotoxic subsets decreased from 78.4% to 59.1% and expression of NKG2D, DNAM-1 and NKP30/NKP46 was also reduced (Table 5). Without being bound by theory, it may be that after the NK cells have effected a change in the cytokine profile in the plasma the NK cells may go from an active state to an exhausted or resting state. This indicates that the batches of allogeneic NK cells are functional.

**Table 5: Change of NK cell product after co-culture with patient's blood serum**

| **Sample** | | **NK1** | **NK2** | **NK3** |
|---|---|---|---|---|
| **Cytotoxic subset (CD56+CD16+)** | Pre-treatment | 78.4 | 90.9 | 67 |
| | Post-treatment | 59.1 | 28.3 | 35.9 |
| **Negative subset (CD56-CD16-)** | Pre-treatment | 12.8 | 6.04 | 25 |
| | Post-treatment | 29.1 | 63.5 | 49 |
| **DNAM-1** | Pre-treatment | 76.7 | 90.6 | 64.9 |
| | Post-treatment | 61.8 | 35.5 | 48.5 |
| **NKG2D** | Pre-treatment | 84.3 | 89.2 | 76.2 |
| | Post-treatment | 57.2 | 18.3 | 43.1 |
| NKP30/NKP46 | Pre-treatment | 81.7 | 92.7 | 61 |
| | Post-treatment | 64.5 | 34.8 | 44.3 |

### Example 3

Exemplary protocol for collection, expansion and preparation of NK cells.

50 ml of peripheral blood from healthy donors is collected in sterile vacutainer tubes containing the anticoagulant (Na-heparin). Plasma is collected for subsequent culture. Peripheral blood mononuclear cell (PBMC) are isolated using Ficoll-Paque^{™} (Cytiva).

The NK expansion is started with 1.5-4^{∗}10⁶ PBMCs with anti-CD16, 10% autologous plasma, IL-15 and IL-2, but without feeder cells. The cell culture density is adjusted to 1.5^{∗}10⁶/ml by X-VIVO 15 medium in T75 flasks. After 3 days culture, X-VIVO 15 medium, autologous plasma and IL-2 is added.

IL2 and X-VIVO 15 medium (Lonza) are added on day 5, and cell density is adjusted to 1.0^{∗}10⁶/ml.

The proportion of NK cells (CD3-CD56+ cells) is tested on day 6 with flow cytometry. If the proportion of NK cell subsets exceeds 10% on day 6, the cultivation is continued, otherwise the culture is considered to have failed and is discarded.

IL2 and RPMI1640 medium is added on day7. Cells are transferred into a culture bag, and cell density is adjusted to 0.7^{∗}10⁶/ml. IL2 and RPMI1640 media are added continuously from day 9 to day 12. Clumping of cells is avoided.

The proportion of NK cells (CD3-CD56+ cells) and the expression level of CD16 on NK cells is assessed on day 13. If the proportion of NK cells is more than 70% and the CD16 positive rate of NK cells is more than 80%, the cells will be tested for sterility and harvested.

The cells are washed twice with normal saline and then transferred into normal saline bag with 5% human serum albumin.

### References

Björkström et al. (2010). CD56 negative NK cells: origin, function, and role in chronic viral disease. Trends in Immunology, 31(11), 401-406.
Chousterman et al. (2017). Cytokine storm and sepsis disease pathogenesis. Semin Immunopathol, 39, 517-528. doi:https://doi.org/10.1007/s00281-017-0639-8
Geller et al. (2011). Use of allogeneic NK cells for cancer immunotherapy. Immunotherapy, 3(12)*.*
Lim et al. (2015, June 3). Present and future of allogeneic natural killer cell therapy. Frontiers in Immunology, 6(Article 286).

## Claims

1. A method for treatment of sepsis in a subject comprising administering a pharmaceutical composition comprising an effective amount of allogeneic Natural Killer cells to said subject.

2. The method according to claim 1, wherein the administering of allogeneic Natural Killer cells is performed through multiple administrations.

3. The method according to any one of claims 1-2, wherein the allogeneic Natural Killer cells are administered by at least one intravenous infusion.

4. The method according to any one of claims 1-3, wherein at least 10⁹ allogeneic Natural Killer cells are administered per administration.

5. The method according to any one of claims 1-4, wherein the subject is immunocompromised.

6. The method according to any one of claims 1-5, wherein the subject suffers from sepsis caused by a microbial infection.

7. The method according to any one of claims 1-6, wherein at least 70% of the cells in the pharmaceutical composition are CD3⁻ and CD56⁺.

8. The method according to any one of claims 1-7, wherein at least 70% of the cells in the pharmaceutical composition are CD56⁺ and CD16⁺.

9. Allogeneic Natural Killer cells for use in a method according to any one of claims 1-8.

10. Use of allogeneic Natural Killer cells in the manufacture of a pharmaceutical composition for use in a method according to any one of claims 1-8.

11. A method for predicting a subject's susceptibility to a method of treatment according to any one of claims 1-6, comprising co-incubating serum from the subject with allogeneic Natural Killer cells and monitoring the level of interleukin-6 in said serum over time, wherein a reduction of the level of interleukin-6 over time is indicative of the subject's susceptibility to the method of treatment.

12. A method for predicting a subject's susceptibility to a method of treatment according to any one of claims 1-6, comprising co-incubating serum from the subject with allogeneic Natural Killer cells and measuring proportional expression of at least one cell surface marker protein on the cell surface of said allogeneic Natural Killer cells before and after co-incubation with the serum, wherein the at least one cell surface marker protein is selected from the group consisting of CD56, CD16, DNAM-1, NKG2D, and NKP30/NKP46, and wherein a reduced proportional expression of the at least one cell surface marker protein on the cell surface of said allogeneic Natural Killer cells is indicative of the subject's susceptibility to the method of treatment.

13. The method according to claim 11 for predicting a subject's susceptibility to a method of treatment, further comprising the steps of the method according to claim 12.

14. The method according to any one of claims 11-13, wherein the serum and the allogeneic Natural Killer cells are co-incubated for a period of time of 16-24 hours.
